# EUROPEAN PATENT SPECIFICATION

(11) **EP 4 031 215 B1**
(45) Date of publication and mention of the grant of the patent: **09.04.2025**
(21) Application number: 20797841.2
(22) Date of filing: 21.09.2020
(51) Int. Cl.: A61M 11/00, A61M 5/31, A61M 5/36

(54) **ATOMIZER DEVICE AND METHOD FOR PREPARATION THEREOF, AND A CONTAINER THEREFOR**
ZERSTÄUBER UND VERFAHREN ZUR HERSTELLUNG DAVON, UND BEHÄLTER DAFÜR
DISPOSITIF D'ATOMISEUR ET SON PROCÉDÉ DE PRÉPARATION, ET RÉCIPIENT POUR CELUI-CI

(30) Priority: 20.09.2019 NL 2023873
(43) Date of publication of application: 27.07.2022
(73) Proprietor: Medspray B.V., 7521 PV Enschede (NL)
(72) Inventor: DE KRUIJF, Wilhelmus Petrus Johannes, 7521 PV Enschede (NL); VAN RIJN, Cornelis Johannes Maria, 7521 PV Enschede (NL); NIJDAM, Wietze, 7521 PV Enschede (NL); VAN EGMOND, Henri Joseph, 7521 PV Enschede (NL)
(74) Representative: Jilderda, Anne Ayolt
(86) International application number: PCT/IB2020/058772
(87) International publication number: WO 2021/053637

(56) References cited:
- EP-A1- 2 011 467
- WO-A1-2009/077091
- US-A- 5 531 119

## Description

The present invention relates to an atomizer device, comprising a container with a liquid chamber for receiving a liquid for atomizing, wherein the liquid chamber comprises an outlet to which a spray nozzle, which is able and configured to form a mist from the liquid under an increased operating pressure, is coupled downstream, wherein the liquid chamber comprises upstream a plunger which is received movably therein and is able and configured to expel during an axial displacement at least a part of the liquid from the liquid chamber, and wherein the container can at least be coupled to actuator means intended and configured to impart an axial displacement to the plunger when actuated. The invention further relates to a method for preparing an atomizer device according to one or more of the preceding claims, wherein a container for receiving a liquid for atomizing is brought into open communication with a spray nozzle downstream, a liquid chamber in the container is filled at least partially with the liquid and the liquid chamber is closed with a plunger movable therein.

This is particularly an atomizer device for pulmonary administration of a pharmaceutical substance. An application is for instance the administration of a substance for pulmonary arterial hypertension (PAH). In pulmonary arterial hypertension the blood vessels in the lungs are restricted, whereby the pressure in those vessels rises. This makes it more difficult for the heart to pump blood around, which has a number of consequences in the body. The higher the pressure in the vessels of the lungs, the more difficult things are for the heart. In a healthy person the pressure in the pulmonary artery is about 14 mm Hg, in someone with pulmonary arterial hypertension it is usually around 25 mm Hg or even higher. The first symptoms of pulmonary arterial hypertension are usually severe fatigue and difficulty breathing, especially during physical exertion. In an advanced stage of the illness, the symptoms are so serious that daily tasks such as getting dressed or showering are already too strenuous. Pulmonary arterial hypertension (PAH) is a chronic illness which is rare but serious. Although the illness can be treated, pulmonary arterial hypertension cannot be cured at present. The disease is a so-called progressive disease, which means that the physical complaints become progressively worse. This is why treatment usually mainly involves temporary combating or suppression of the symptoms.

Loaded with a suitable pharmaceutical, an atomizer device in particular can provide rapid and immediate relief in the event of a sudden attack. This is because the substance can in that case be administered in pulmonary manner, and thus the absorbed directly via the lungs into the blood vessels of the lungs. Treprostinil is a drug which is prescribed for the treatment of pulmonary arterial hypertension. The substance dilates the blood vessels and lowers the blood pressure in the vessels of the lungs. It also inhibits the formation of blood clots. Treprostinil is a synthetic analog of prostacyclin. Pulmonary administration causes relatively little stress for the user, and has a fast action.

With a view to pulmonary administration of Treprostinil an atomizer device can be filled with a solution of this substance, corresponding to a number of doses. The user can self-administer the substance as soon as the need therefor arises, for instance when shortness of breath occurs suddenly. It is important here that an accurately prescribed quantity of the substance is always dispensed per treatment in order to ensure efficaciousness. An accurately controlled dosing per treatment is usually also of great importance with other pharmaceutical substances for pulmonary administration.

A known device of the type described in the preamble is for instance known from the European patent application EP 2.485.793. This known device comprises a container which is filled with a liquid pharmaceutical and is then closed with the stopper. By imparting a controlled stroke to the stopper a volume corresponding therewith is expelled from the container and forced under increased pressure to the spray nozzle. The aim is thus to dispense an accurate dose of the substance in the form of a mist, so that it can be inhaled.

The operation of the known device is however disrupted in that air and air bubbles can be enclosed in the liquid during filling of the container. After the plunger has been arranged, they stay in the liquid. An air bubble in the expelled volume however causes an error in the dispensed dose, whereby too little product may unintentionally be dispensed. WO2009077091 describes an injection device having a membrane 8 in the housing to make air bubbles escape.

The present invention has for its object, among others, to provide an atomizer device and method for manufacture thereof which allows for an accurate, precisely defined dosing.

In order to achieve the intended object an atomizer device of the type described in the preamble has the feature according to the invention that provided between the plunger of the liquid chamber and the spray nozzle is an air bubble barrier which, from a side thereof facing toward the plunger, is at least substantially unbridgeable to possible air bubbles in the liquid, but which comprises a passage for the purpose of allowing at least substantially free passage of the liquid. The air bubble barrier received here according to the invention between the plunger and the spray nozzle prevents an air bubble undesirably enclosed upstream from being able to migrate therethrough from a side thereof facing toward the plunger. Instead, the air bubble is stopped by the barrier. As a result hereof, only liquid is transported through the device upon a stroke by the plunger, so that a volume expelled by the plunger actually corresponds with a volume of liquid which is supplied to the spray nozzle and escapes as a mist. This enables an accurate dosing of the liquid, despite the possible presence of air bubbles in the liquid.

A particular embodiment of the atomizer device has the feature according to the invention that at least one of an inner wall of the container and an outer wall of the air bubble barrier is hydrophobic adjacently of an inlet of the passage of the air bubble barrier. Selectivity between passage of liquid relative to an air bubble is controlled mainly by a surface tension and an interaction of the two with the material of the air bubble barrier and of the environment. This selectivity can be enhanced by creating a hydrophobic environment upstream, in which possible air bubbles will be trapped and held.

Conversely or additionally, a further particular embodiment of the atomizer device has the feature according to the invention that the passage comprises at least one liquid channel extending through the barrier from upstream to downstream, wherein at least a wall of the channel is hydrophilic. The liquid channel with a hydrophilic surface here conversely provides an attraction for the liquid, while the surface tension of an air bubble will resist entering.

On one side the liquid channel is so narrow that a surface tension of a possible air bubble resists entering, while on the other the liquid can nevertheless enter freely. In this respect a specific embodiment of the atomizer device has the feature according to the invention that the passage comprises a liquid channel which extends through the barrier from upstream to downstream and which opens upstream with an inlet having a cross-section smaller than 2000 micron and greater than 50 micron.

Said cross-section has particular effect at an inlet of the liquid channel in preventing entry of air bubbles. In order to nevertheless keep a flow resistance of the channel acceptably low a further preferred embodiment of the device has the feature according to the invention that the liquid channel widens, particularly gradually, in downstream direction and opens downstream with a cross-section greater than a cross-section of the inlet on an opposite outer end of the liquid channel.

The air bubble barrier is preferably provided adjacently of, at least close to, the outlet of the liquid chamber. Practically all liquid and air bubbles possibly present therein will in that case be situated upstream of the barrier so that all air bubbles therein are no longer able to freely migrate to the spray nozzle. A preferred embodiment of the device according to the invention in this respect has the feature that the air bubble barrier is formed by a separate barrier device, wherein the passage comprises a continuous liquid channel which resists entry of air bubbles but opens both upstream and downstream in order to form a passage for liquid. Such an individual barrier device can be inserted in the liquid chamber before filling the liquid chamber with liquid and arranging the plunger. The barrier device is however preferably provided downstream of the outlet, externally of the liquid chamber, so that the liquid chamber can be filled irrespective of a (presence of a) barrier device. This has in practice been found of great significance from a logistical viewpoint.

The barrier device can comprise an assembly composed of separate components. A further preferred embodiment of the atomizer device nevertheless has the feature according to the invention that the barrier device is formed at least substantially by a monolithic barrier body in which the liquid channel extends. The barrier device is thus formed at least substantially integrally; for instance by thermoforming from a suitable plastic, particularly by injection moulding. In a specific embodiment the atomizer device according to the invention is here characterized in that the barrier body comprises an at least substantially solid body, particularly a plastic body, more particularly a plastic body which is hydrophilic at least in the channel. Such an integral barrier requires no further assembly steps and can otherwise also be realized at a relatively low cost price.

Particularly advantageous results have been achieved in practice with a particular embodiment of the atomizer device according to the invention which is characterized in that the barrier device comprises a spout in which the liquid channel extends, wherein a downward directed side piece extends from the spout and extends over only a limited part of a length of the spout, and here maintains a distance from the spout. This distance provides an intermediate space between the spout and the side piece or an inner wall of the container against which the side piece lies sealingly.

Due to a difference in density, air bubbles naturally tend to float on the liquid. During migration to the outlet, air bubbles will therefore mainly approach the spout with the passage eccentrically and pass beyond the inlet of the spout. These air bubbles can then nestle in this intermediate space between the spout and the side piece or an inner wall of the container. On one hand they adhere here to an inner wall of the liquid chamber, on the other a liquid flow resulting from a stroke of the plunger resists escape from said intermediate space.

Deflection of possible air bubbles to a position beyond the inlet of the passage is enhanced further in a further preferred embodiment of the device according to the invention, which is characterized in that a screen is provided upstream close to an inlet of the liquid channel, which screen shields the inlet of the liquid channel from air bubbles but allows space for passage of the liquid laterally. The screen can here form part of the barrier device and particularly be formed integrally therewith. It is however also possible to add the screen as a separate component. The shielding of the screen consists mainly of the screen imposing on a possible air bubble an unnatural trajectory, which is unachievable in practice, toward the inlet, whereby it is guided away from the inlet of the passage and nestles laterally of the barrier device. Liquid is however not or hardly impeded by the screen which is placed at a distance from the inlet and leaves space laterally. An additional barrier to possible air bubbles is thus formed thereby.

Instead of a barrier device with one or more passages in the form of one or several liquid channels, many alternatives are possible for the barrier device. A further particular embodiment of the device thus has the feature according to the invention that the barrier device comprises a sponge body with at least one liquid channel therein, which at least one liquid channel opens both upstream and downstream. Such a sponge body has an open labyrinth of liquid channels which does not form a barrier to the liquid, or hardly so, but is inaccessible or at least unbridgeable to air bubbles. Use is particularly made here of a hydrophilic sponge body, which has this effect to an increased extent.

A further particular embodiment of the device according to the invention is characterized in that the barrier device comprises a grid with a system of openings which open both upstream and downstream and each have a cross-section of less than 100 micron. The openings in the grid are here dimensioned such that the surface tension of an air bubble loses out against that of the liquid, whereby the openings fill exclusively with liquid. A hydrophilic grid, which has this effect to an increased extent, is particularly applied here.

From a viewpoint of availability of parts, a further particular embodiment of the device has the feature according to the invention that the container comprises a cylindrical liquid chamber and is particularly interchangeable with a standard barrel of a medicinal syringe. Such a container is for instance manufactured from a medical grade plastic or glass, and as such can be used directly in the device. The container can here optionally take a removable and exchangeable form, making the device refillable.

Various measures can be applied in order to prevent or at least counter inclusion of air as far as possible. In a particular embodiment the device is characterized for this purpose in that an at least temporarily air-permeable plunger is applied, for instance in that the plunger comprises a liquid-tight but air-permeable membrane or in that the plunger comprises an air channel which was closed liquid-tightly. This achieves that air which may be enclosed by the plunger can still escape via the plunger, rather than dissolving in the liquid. The formation of air bubbles is thus effectively prevented.

Air which is initially located downstream of the air bubble barrier will not be hindered thereby from that position. In order to prevent such a possible downstream air inclusion from eventually also reaching the spray nozzle and interfering with the dosing of the device, a method for preparing an atomizer device of the type described in the preamble has the feature according to the invention that, before arranging the plunger, a barrier device is placed upstream of the spray nozzle, which barrier device is at least substantially unbridgeable from a side thereof device facing toward the plunger to possible air bubbles in the liquid but which comprises a passage for at least substantially free passage of the liquid, and that after the plunger was arranged possible air bubbles in the liquid are driven to the side of the barrier device facing toward the plunger. By thus displacing air which may reside downstream during or after manufacture to a position upstream of the air bubble barrier, this air will then also be stopped thereby.

Such an upstream displacement of air which may be enclosed can be performed in various ways. A particularly practical method, which also leaves an integrity and possible sterility of the content of the device intact, has the feature according to the invention that the device is subjected to a centrifuge after the plunger was arranged in order to drive possible air bubbles in the liquid to the side of the barrier device facing toward the plunger.

A preferred embodiment of the method according to the invention is here characterized in that the barrier device is arranged before the liquid chamber is filled with the liquid. All liquid is thus situated upstream from the barrier ab initio, so that all possible air bubbles in the liquid will also be situated upstream of the barrier. Only a possible air remainder that was enclosed downstream need then still be driven to the other side of the barrier.

The invention also relates to a container for application in the above described device, and will now be further elucidated with reference to a drawing. In the drawing:
- Figure 1: shows a perspective view of an exemplary embodiment of an atomizer device according to the invention;
- Figure 2: shows a cross-section of the device of figure 1;
- Figure 3: shows a perspective view of a container with spray nozzle as applied in the container of figure 2;
- Figure 4: shows a cross-sectional view of the container of figure 3 provided with a barrier device according to the invention;
- Figure 5: shows a perspective and partially cross-sectional view of the container of figure 3 provided with a barrier device according to the invention;
- Figure 6: shows a cross-section of the barrier device as applied in the container of figures 4 and 5;
- Figure 7: shows a perspective view of the barrier device as applied in the container of figures 4 and 5;
- Figure 8: shows a perspective view of a first alternative barrier device as applicable in the container of figures 4 and 5;
- Figure 9: shows a perspective view of a second alternative barrier device as applicable in the container of figures 4 and 5;
- Figure 10: shows a cross-sectional view of a second exemplary embodiment of a container with spray nozzle for an atomizer device according to the invention;
- Figure 10A: shows a cross-sectional view of the barrier device as applied in the container of figure 10;
- Figure 11: shows a cross-section of an alternative barrier device as applicable in the container of figure 10;
- Figure 12: shows a cross-section of the adapter body with the barrier device as applied in the container of figure 10;
- Figure 13: shows a test result of a repeated dispensing per dose of an atomizer device without air bubble barrier; and
- Figure 14: shows a test result of a repeated dispensing per dose of an atomizer device according to the invention which is provided with an air bubble barrier (device).

It is otherwise noted that the figures are purely schematic and not always drawn to (the same) scale. Some dimensions in particular may be exaggerated to greater or lesser extent for the sake of clarity. Corresponding parts are designated in the figures with the same reference numeral.

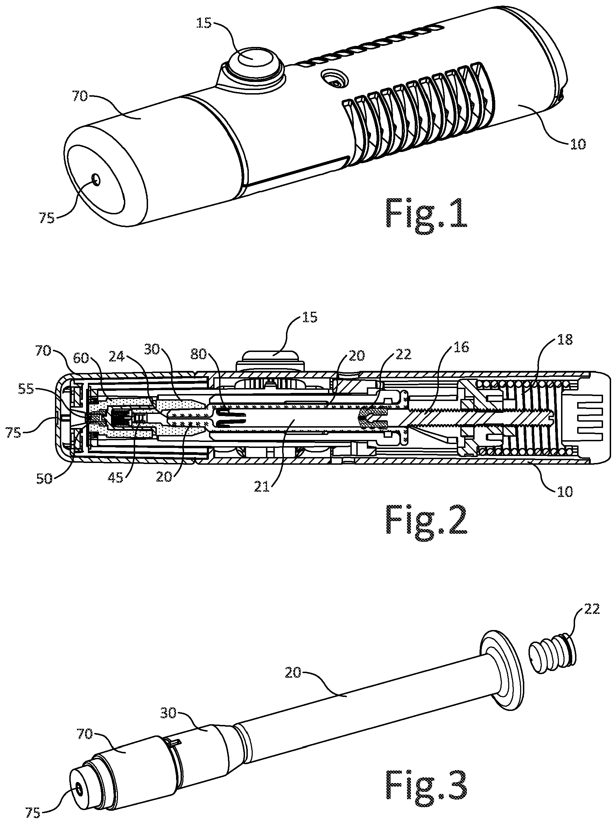

Figures 1 and 2 show an exemplary embodiment of an atomizer device for atomization and pulmonary administration of a pharmaceutical. In this example this is a device for pulmonary administration of a pharmacological solution of Treprostinil. Treprostinil is a drug prescribed for the treatment of pulmonary arterial hypertension. The substance dilates the blood vessels and lowers the blood pressure in the vessels of the lungs. It also inhibits the formation of blood clots. So far, this substance has mostly been administered subcutaneously or intravenously. Pulmonary administration it is significantly less stressful for the user. The device described here provides the user with the option of self-administration. For this purpose the device comprises a push button 15 whereby, when actuated, actuator means in the form of an internal spring 18 are slackened over a predetermined length so that, without further intervention by the user, a precisely determined dose of the substance is dispensed.

The device is composed substantially of plastic components. The device is bounded externally by a plastic housing 10 which accommodates the spring mechanism 18 and a container 20 with a cylindrical liquid chamber 21 in which the substance for dispensing is received. The container comprises here a glass tube, similar to the barrel of a standard syringe, and has a volume in the order of for instance ½, 1, 1½ or 2 millilitres. In this example use is made of a chamber 21 with a maximum volume of 1 millilitre. On a rear side (i.e. upstream) chamber 21 is closed with a plunger 22, while liquid chamber 21 debouches downstream in an outlet 24. Plunger 22 comprises for instance a synthetic rubber and is received movably in liquid chamber 21. A valve housing 40 with a spring-loaded valve 45 and a spray nozzle 50 with a spray body 55 are mounted successively on the container on the outlet side 24. Spring-loaded valve 45 opens at a minimum operating pressure in the order of 20 bar and thus ensures that the liquid is always supplied to spray nozzle 50 at such a minimum operating pressure. Situated in spray nozzle 50 is a spray body 55 which is provided with one or more spray openings having dimensions ranging from sub-micron to several micrometres, from which the liquid escapes at the stated operating pressure in the form of a jet of successive droplets so as to form a mist. The whole is closed with a plastic cover 70 which connects flush with the housing 10 of the device and which is provided centrally with a spray hole 75.

For the transition from glass container 20 to plastic valve housing 40 of spring-loaded valve 45 use is made of an adapter 30 which provides on the side of container 20 a so-called Luer taper for the purpose of a leak-tight connection. Valve housing 40 is in turn received liquid-tightly in a spray nozzle adapter 60 with spray opening 65, which also comprises the spray head 50 with the spray body 55. The container is filled with a precisely measured quantity of about 150 microlitres of the substance for atomizing; enough for three doses in the order of 35 microlitres after so-called priming of the device is done once or twice in order to expel air which may initially be present downstream.

The metal coil spring 18 lies under bias and engages with a spindle 16 on the plunger 22, which makes a fixed number of revolutions in a slackening of spring 18 caused by push button 15.

Multiplied by a pitch of the spindle 16, this corresponds to a precisely determined stroke of spindle 16 and thereby a precisely imparted stroke of plunger 22 in the chamber 21 of tube 20. This in turn corresponds with a substantially precisely determined volume (dose) of liquid being expelled from container 20. As such, the device is configured and dimensioned such that such a shot dosage can thus be dispensed four or five times in succession, with each actuation of push button 15.

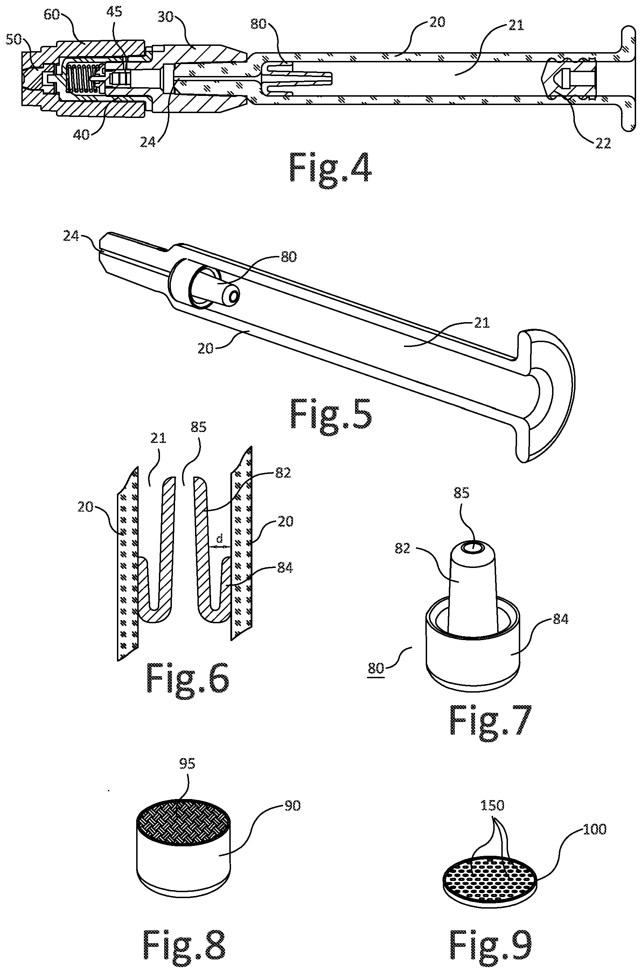

Although a volume expelled from liquid chamber 21 can per se be controlled well by means of a stroke which can be precisely imparted to plunger 22, this need not always correspond with an actually dispensed dose of the substance. Trapped air and air bubbles in particular interfere with an accurate dosing due to the volume which is taken up thereby but is in fact empty. In order to prevent this the device is actuated once or twice before use in order to expel air downstream of the container and out of the device. This so-called priming of the device is repeated until liquid appears at spray opening 75. According to the invention, air bubbles which may be enclosed in the liquid further upstream are stopped by means of an air bubble barrier 80 provided for this purpose between spray nozzle 50 and plunger 22. In this example this air bubble barrier comprises a separate barrier device 80 which is arranged in liquid chamber 21 just in front of outlet 24. This device is shown in further detail in cross-section in figure 6 and in perspective view in figure 7.

Barrier device 80 comprises centrally a hollow spout 82 in which a liquid channel 85 extends continuously. A downward directed side piece 84 extends integrally from the spout from an outlet side of the spout. This side piece 84 lies fittingly against a wall 20 of liquid chamber 21 and thereby provides an airtight seal. Side piece 84 extends over only a limited part of a length of spout 82, whereby upstream a certain distance d is kept to wall 20 of liquid chamber 21 and to spout 82. This distance d forms an intermediate space 86 in which possible air bubbles become trapped if they migrate along with the flow of the liquid through chamber 21.

With a cross-section in the order of 1 millimetre, the passage 85 through spout 82 is so narrow that air bubbles are practically unable to enter it. In order to keep a flow resistance through barrier device 80 sufficiently low the channel 85 can be embodied with an optionally gradually increasing cross-section from the upstream inlet thereof. In the shown example this has been applied in that channel 85 narrows more or less conically toward that inlet.

Air bubbles will be displaced at the inlet of channel 85 by the liquid which can here freely enter channel 85 and pass the barrier. Contributing hereto is the fact that at least an inner wall of liquid channel 85 is hydrophilic. For this purpose a hydrophilic coating can be applied or, as in this case, the whole barrier device can be manufactured from hydrophilic material, particularly a hydrophilic plastic such as polyethylene.

Any air bubbles are thereby conversely prevented from entering channel 85 and instead will be displaced laterally of spout 82, into the intermediate space 86 formed there with the wall 20 of liquid chamber 21, and be trapped therein. This is further contributed to by a hydrophobic material of container 20 or a hydrophobic coating on the inner wall thereof, at least adjoining barrier device 80.

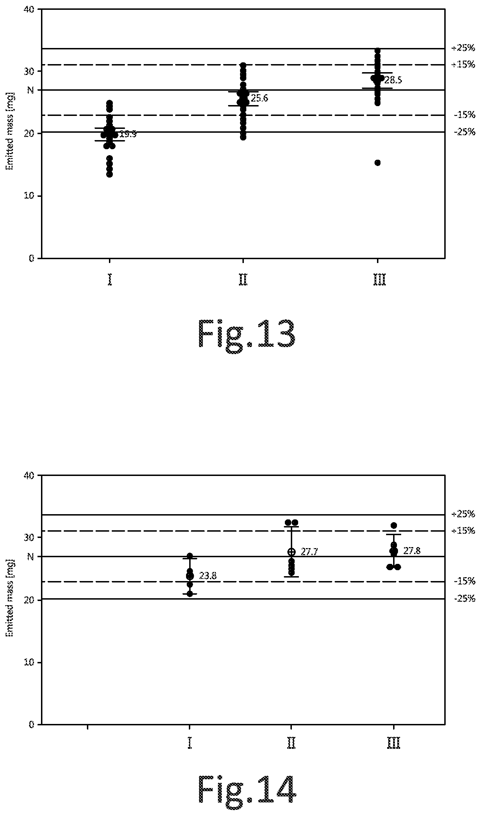

Only liquid thus emerges at the outlet 24 of container 20, so that a stroke of plunger 22 and the volume displaced thereby can actually be equated substantially one-on-one with a dispensed dose of the substance. Figures 13 and 14 clearly show the operation of the air bubble barrier according to the invention. Figure 13 shows graphically the measured quantity of three subsequent dosages I-III with the device, without the shown barrier device being applied therein. The experiment was repeated a number of times here in order to determine a significant average value per individual dosage. In the figure, these average values are stated for each dosage I-III. The object is to dispense a standard dosage N of about 27 mg, wherein from a clinical viewpoint a deviation of 25% is permitted per individual dosage and the average within a group I-III must lie within 15% of that standard. This is shown in the figure with respective horizontal boundary lines ±25% and ± 15%. It can be seen that particularly the first dosage I falls significantly outside these boundaries, which is attributed to air (bubbles) being pushed out instead of liquid.

In figure 14 the same experiment was performed with a device of figure 1, wherein the barrier device 80 was applied as according to the invention. It can be clearly seen that when barrier device 80 is applied, the three successive dosages I-III are higher and are of a more constant level than when it is not applied. All individual dosages now particularly lie within the bandwidth of ±25% around the standard N, and the average of the dosages per group within the bandwidth of ± 15%.

Figure 8 shows an alternative barrier device 90 which can be applied in the atomizer device in the same way as that of figure 7. In this case the barrier is formed by a sponge body or foam body 95 with an open passage structure through which extend liquid channels which open both upstream and downstream. With an average cross-section in the order of 100 micron, this passage structure is so narrow that air bubbles cannot enter it. In this example the sponge body comprises a hydrophilic foam, for instance of polypropylene or polystyrene, and conversely sucks up the liquid, and so this liquid has free passage.

Instead of such a foam with an open pore structure, it is optionally also possible to apply a grid 100 with a defined system of a large number of narrow passages 150 therein. An example of such a barrier device is shown in figure 9. Use is made of a plastic body of polyethylene which was obtained by extrusion. Openings 150 were imparted here by the mould used in extrusion, after which the body 100 was separated from a greater whole by sawing or cutting. Instead of this, it is also possible to apply a solid disc in which the openings 150 are provided in mechanical manner (perforation) or chemical manner (etching). Use is here preferably also made of a hydrophilic body 100, at least a body that was provided with a hydrophilic coating. The openings typically have a cross-section in the order of between 30 and 100 micron.

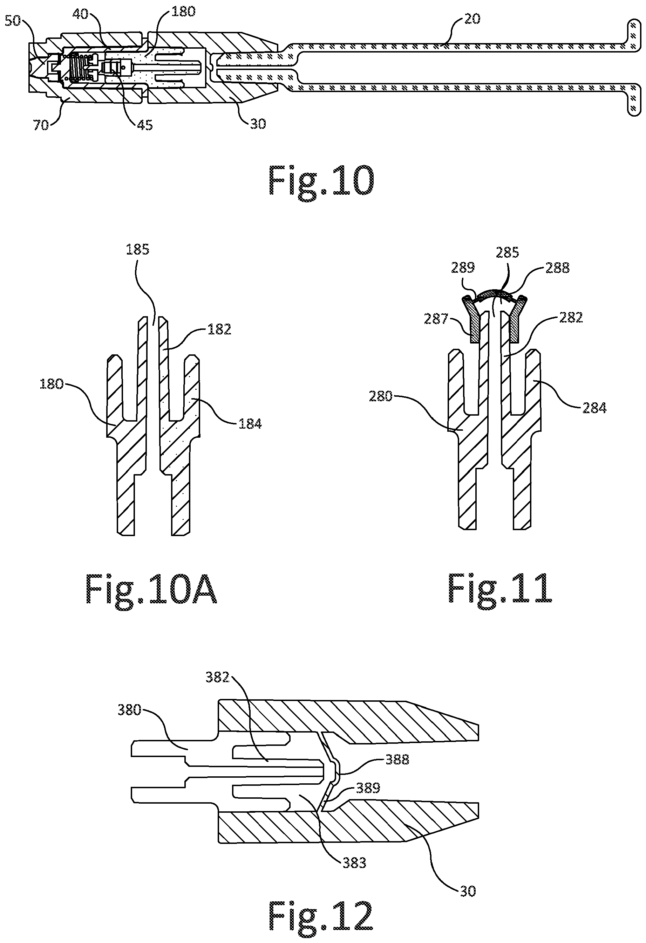

Figure 10 provides an example of a second embodiment of a container with spray nozzle for an atomizer device according to the invention. This embodiment differs from that of figure 4 in that a barrier device 180 is in this case provided outside container 20 in downstream direction. In this case the barrier device is also formed by a uniform barrier body 180 which is shown enlarged and in more detail in figure 10A. The barrier body comprises a central spout with therein a narrow passage 185 which prevents possible air bubbles from continuing their way further downstream. Instead, such air bubbles are carried away laterally and trapped between spout 182 and an inner wall of adapter body 30. For this purpose the passage 185 has a cross-section in the order of 1000 micron. A side piece 184 directed downward over a part of a length of spout 182 provides for a practically seamless fit between barrier body 180 and said inner wall of adapter body 30, so that air bubbles are unable to pass. The barrier body is here advantageously also manufactured from a hydrophilic plastic. Because the barrier body is in this case also provided between the plunger 22 of container 20 and the spray nozzle 50, it is prevented that air in the form of air bubbles which may become enclosed during filling of container 20 is able to reach the spray nozzle and interfere with a precise dosing.

An alternative barrier body 280 is shown in figure 11 and, if desired, can be utilized instead of that of figure 10A in the device of figures 4 or 10. In this case a deflector is additionally provided on the barrier body upstream of an inlet of passage 285 in order to guide air bubbles which may be migrating to passage 285 away therefrom. In the embodiment of figure 12 the deflector 288 is formed by a hat 287 which is provided on the spout, in this example as a separate component. The deflector is formed by a central screen 288 in the axis of passage 285, which is suspended by means of a set of arms 289. Provided between the arms 289 of screen 288 are passages which allow for free inflow of liquid to passage 285. Screen 288 extends over at least substantially a whole active section of passage 285 in order to thus wholly shield it from air bubbles. A slightly convex or chamfered roof of screen 288 enhances the deflecting function of the screen and guides air bubbles to the side; away from the inlet of passage 285, where they will nestle between the spout and the wall of the container without interfering with a precise dosing of liquid mist by the device. In this case a downward directed side piece or shoulder 284 also provides for a fit against the inner wall of liquid chamber 20 or adapter 30.

As shown in figure 11, such a screen 289 can be provided, optionally integrally, on the spout 282 of the barrier body, but can also be provided in the container upstream thereof, separate from the barrier. Figure 12 provides an example of this. In this case a screen 388 is formed integrally with the adapter body 30. It is suspended therein by means of a set of arms 389 which leave free a space therebetween for the purpose of free passage of liquid. Screen 388 however guides possible air bubbles away from passage 385 in the air bubble barrier 380 which is placed a short distance from the screen. A space between a central spout 382 of the barrier and a wall of adapter body 30 forms a chamber 383 in which these possible air bubbles are trapped and held, while a downward directed side piece or shoulder provides for a fit against the inner wall of adapter body 30.

Although the invention has been further elucidated above on the basis of only a single exemplary embodiment, it will be apparent that the invention is by no means limited thereto. On the contrary, many variations and embodiments are still possible within the scope of the invention for a person with ordinary skill in the art.

## Claims

1. Atomizer device, comprising a container with a liquid chamber for receiving a liquid for atomizing, wherein the liquid chamber comprises an outlet to which a spray nozzle (50), which is able and configured to form a mist from the liquid under an increased operating pressure, is coupled downstream, wherein the liquid chamber (21) comprises upstream a plunger (21) which is received movably therein and is able and configured to expel during an axial displacement at least a part of the liquid from the liquid chamber, and wherein the container can at least be coupled to actuator means intended and configured to impart an axial displacement to the plunger when actuated, **characterized in that** provided between the plunger of the liquid chamber and the spray nozzle is an air bubble barrier (80) which, from a side thereof facing toward the plunger, is at least substantially unbridgeable to possible air bubbles in the liquid, but which comprises a passage for the purpose of allowing at least substantially free passage of the liquid.

2. Atomizer device according to claim 1, **characterized in that** the passage comprises at least one liquid channel (85) extending through the air bubble barrier from upstream to downstream, wherein at least a wall of the channel is hydrophilic.

3. Atomizer device according to claim 1 or 2, **characterized in that** at least one of an inner wall of the container and an outer wall of the air bubble barrier is hydrophobic adjacently of an inlet of the passage of the air bubble barrier.

4. Atomizer device according to one or more of the preceding claims, **characterized in that** the passage comprises a liquid channel which extends through the barrier from upstream to downstream and which opens upstream with an inlet having a cross-section smaller than 2000 micron and greater than 50 micron.

5. Atomizer device according to claim 4, **characterized in that** the liquid channel widens, particularly gradually, in downstream direction and opens downstream with a cross-section greater than a cross-section of the inlet on an opposite outer end of the liquid channel.

6. Atomizer device according to one or more of the preceding claims, **characterized in that** the air bubble barrier is formed by a separate barrier device, wherein the passage comprises a continuous liquid channel which resists entry of air bubbles but opens both upstream and downstream in order to form a passage for liquid.

7. Atomizer device according to claim 6, **characterized in that** the barrier device is formed at least substantially by a monolithic barrier body in which the liquid channel extends.

8. Atomizer device according to claim 7, **characterized in that** the barrier body comprises an at least substantially solid body, particularly a plastic body, more particularly a plastic body which is hydrophilic at least in the liquid channel.

9. Atomizer device according to one or more of the claims 6, 7 or 8, **characterized in that** the barrier device comprises a spout in which the liquid channel extends, wherein a downward directed side piece extends from the spout and extends over only a limited part of a length of the spout, and here maintains a distance from the spout.

10. Atomizer device according to claim 9, **characterized in that** a screen is provided upstream close to an inlet of the liquid channel, which screen shields the inlet of the liquid channel from air bubbles but allows space for passage of the liquid laterally.

11. Atomizer device according to claim 6, **characterized in that** the barrier device comprises a sponge body with at least one liquid channel therein, which at least one liquid channel opens both upstream and downstream.

12. Atomizer device according to claim 6, **characterized in that** the barrier device comprises a grid with a system of openings which open both upstream and downstream and each have a cross-section of less than 100 micron.

13. Atomizer device according to one or more of the preceding claims, **characterized in that** the container comprises a cylindrical liquid chamber and is particularly interchangeable with a standard barrel of a medicinal syringe.

14. Atomizer device according to one or more of the preceding claims, **characterized in that** the plunger comprises a liquid-tight but air-permeable membrane.

15. Atomizer device according to one or more of the preceding claims, **characterized in that** the plunger comprises an air channel which was closed liquid-tightly.

16. Container with an optionally filled liquid chamber provided with an air bubble barrier for application in the atomizer device according to one or more of the preceding claims.

17. Method for preparing an atomizer device according to one or more of the preceding claims, wherein a container for receiving a liquid for atomizing is brought into open communication with a spray nozzle downstream, a liquid chamber in the container is filled at least partially with the liquid and the liquid chamber is closed with a plunger movable therein, **characterized in that**, before arranging the plunger, a barrier device is placed upstream of the spray nozzle, which barrier device is at least substantially unbridgeable from a side thereof device facing toward the plunger to possible air bubbles in the liquid but which comprises a passage for at least substantially free passage of the liquid, and that after the plunger was arranged possible air bubbles in the liquid are driven to the side of the barrier device facing toward the plunger.

18. Method according to claim 17, **characterized in that** the device is subjected to a centrifuge after the plunger was arranged in order to drive possible air bubbles in the liquid to the side of the barrier device facing toward the plunger.

19. Method according to claim 17 or 18, **characterized in that** the barrier device is arranged before the liquid chamber is filled with the liquid.

## Patentansprüche

1. Zerstäubervorrichtung, umfassend einen Behälter mit einer Flüssigkeitskammer zur Aufnahme einer zu zerstäubenden Flüssigkeit, wobei die Flüssigkeitskammer einen Auslass aufweist, an den eine Sprühdüse (50), die in der Lage und ausgebildet ist, unter einem erhöhten Betriebsdruck einen Nebel aus der Flüssigkeit zu bilden, stromabwärts gekoppelt ist, wobei die Flüssigkeitskammer (21) stromaufwärts einen Kolben (21) umfasst, der darin beweglich aufgenommen ist und in der Lage und konfiguriert ist, während einer axialen Verschiebung zumindest einen Teil der Flüssigkeit aus der Flüssigkeitskammer auszustoßen, und wobei der Behälter zumindest mit einem Betätigungsmittel gekoppelt werden kann, das dazu bestimmt und konfiguriert ist, dem Kolben bei Betätigung eine axiale Verschiebung zu verleihen,
**dadurch gekennzeichnet, dass** zwischen dem Kolben der Flüssigkeitskammer und der Sprühdüse eine Luftblasenbarriere (80) vorgesehen ist, die von einer dem Kolben zugewandten Seite aus für mögliche Luftblasen in der Flüssigkeit zumindest im Wesentlichen unüberbrückbar ist, die jedoch einen Durchlass aufweist, um einen zumindest im Wesentlichen freien Durchgang der Flüssigkeit zu ermöglichen.

2. Zerstäubervorrichtung nach Anspruch 1, **dadurch gekennzeichnet, dass** der Durchgang mindestens einen Flüssigkeitskanal (85) umfasst, der sich durch die Luftblasenbarriere von stromaufwärts nach stromabwärts erstreckt, wobei mindestens eine Wand des Kanals hydrophil ist.

3. Zerstäubervorrichtung nach Anspruch 1 oder 2, **dadurch gekennzeichnet, dass** mindestens eine Innenwand des Behälters oder eine Außenwand der Luftblasenbarriere in der Nähe eines Einlasses des Durchlasses der Luftblasenbarriere hydrophob ist.

4. Zerstäubervorrichtung nach einem oder mehreren der vorhergehenden Ansprüche, **dadurch gekennzeichnet, dass** der Durchgang einen Flüssigkeitskanal umfasst, der sich durch die Barriere von stromaufwärts nach stromabwärts erstreckt und der stromaufwärts mit einem Einlass mündet, der einen Querschnitt von weniger als 2000 Mikrometer und mehr als 50 Mikrometer aufweist.

5. Zerstäubervorrichtung nach Anspruch 4, **dadurch gekennzeichnet, dass** sich der Flüssigkeitskanal in stromabwärtiger Richtung, insbesondere allmählich, erweitert und stromabwärts mit einem Querschnitt öffnet, der größer ist als der Querschnitt des Einlasses an einem gegenüberliegenden äußeren Ende des Flüssigkeitskanals.

6. Zerstäubervorrichtung nach einem oder mehreren der vorhergehenden Ansprüche, **dadurch gekennzeichnet, dass** die Luftblasenbarriere durch eine separate Barrierevorrichtung gebildet wird, wobei der Durchlass einen kontinuierlichen Flüssigkeitskanal umfasst, der dem Eintritt von Luftblasen widersteht, sich jedoch sowohl stromaufwärts als auch stromabwärts öffnet, um einen Durchlass für Flüssigkeit zu bilden.

7. Zerstäubervorrichtung nach Anspruch 6, **dadurch gekennzeichnet, dass** die Sperrvorrichtung zumindest im Wesentlichen durch einen monolithischen Sperrkörper gebildet wird, in dem sich der Flüssigkeitskanal erstreckt.

8. Zerstäubervorrichtung nach Anspruch 7, **dadurch gekennzeichnet, dass** der Sperrkörper einen zumindest im Wesentlichen festen Körper, insbesondere einen Kunststoffkörper, insbesondere einen zumindest im Flüssigkeitskanal hydrophilen Kunststoffkörper, umfasst.

9. Zerstäubervorrichtung nach einem oder mehreren der Ansprüche 6, 7 oder 8, **dadurch gekennzeichnet, dass** die Sperrvorrichtung eine Tülle umfasst, in der sich der Flüssigkeitskanal erstreckt, wobei sich ein nach unten gerichtetes Seitenteil von der Tülle aus erstreckt und sich nur über einen begrenzten Teil einer Länge der Tülle erstreckt und hier einen Abstand von der Tülle beibehält.

10. Zerstäubervorrichtung nach Anspruch 9, **dadurch gekennzeichnet, dass** stromaufwärts in der Nähe eines Einlasses des Flüssigkeitskanals ein Sieb vorgesehen ist, das den Einlass des Flüssigkeitskanals gegen Luftblasen abschirmt, aber seitlich Raum für den Durchgang der Flüssigkeit lässt.

11. Zerstäubervorrichtung nach Anspruch 6, **dadurch gekennzeichnet, dass** die Sperrvorrichtung einen Schwammkörper mit mindestens einem Flüssigkeitskanal darin umfasst, wobei der mindestens eine Flüssigkeitskanal sowohl stromaufwärts als auch stromabwärts mündet.

12. Zerstäubervorrichtung nach Anspruch 6, **dadurch gekennzeichnet, dass** die Sperrvorrichtung ein Gitter mit einem System von Öffnungen umfasst, die sowohl stromaufwärts als auch stromabwärts münden und jeweils einen Querschnitt von weniger als 100 Mikrometer aufweisen.

13. Zerstäubervorrichtung nach einem oder mehreren der vorhergehenden Ansprüche, **dadurch gekennzeichnet, dass** der Behälter eine zylindrische Flüssigkeitskammer aufweist und insbesondere mit einem Standardzylinder einer medizinischen Spritze austauschbar ist.

14. Zerstäubervorrichtung nach einem oder mehreren der vorhergehenden Ansprüche, **dadurch gekennzeichnet, dass** der Kolben eine flüssigkeitsdichte, aber luftdurchlässige Membran aufweist.

15. Zerstäubervorrichtung nach einem oder mehreren der vorhergehenden Ansprüche, **dadurch gekennzeichnet, dass** der Kolben einen Luftkanal aufweist, der flüssigkeitsdicht verschlossen wurde.

16. Behälter mit einer optional gefüllten und mit einer Luftblasenbarriere versehenen Flüssigkeitskammer zur Verwendung in der Zerstäubervorrichtung nach einem oder mehreren der vorhergehenden Ansprüche.

17. Verfahren zur Herstellung einer Zerstäubervorrichtung nach einem oder mehreren der vorhergehenden Ansprüche, wobei ein Behälter zur Aufnahme einer Flüssigkeit zum Zerstäuben in offene Verbindung mit einer stromabwärts gelegenen Sprühdüse gebracht wird, eine Flüssigkeitskammer in dem Behälter zumindest teilweise mit der Flüssigkeit gefüllt wird und die Flüssigkeitskammer mit einem darin beweglichen Kolben verschlossen wird, **dadurch gekennzeichnet, dass** vor dem Anordnen des Kolbens, eine Sperrvorrichtung stromaufwärts der Sprühdüse angeordnet ist, wobei die Sperrvorrichtung von einer Seite der Vorrichtung, die dem Kolben zugewandt ist, für mögliche Luftblasen in der Flüssigkeit zumindest im Wesentlichen unüberbrückbar ist, aber einen Durchgang für einen zumindest im Wesentlichen freien Durchgang der Flüssigkeit aufweist, und dass nach dem Anordnen des Kolbens mögliche Luftblasen in der Flüssigkeit zu der Seite der Sperrvorrichtung getrieben werden, die dem Kolben zugewandt ist.

18. Verfahren nach Anspruch 17, **dadurch gekennzeichnet, dass** die Vorrichtung einer Zentrifuge unterzogen wird, nachdem der Kolben angeordnet wurde, um eventuelle Luftblasen in der Flüssigkeit auf die dem Kolben zugewandte Seite der Sperrvorrichtung zu treiben.

19. Verfahren nach Anspruch 17 oder 18, **dadurch gekennzeichnet, dass** die Sperrvorrichtung angeordnet wird, bevor die Flüssigkeitskammer mit der Flüssigkeit gefüllt wird.

## Revendications

1. Dispositif d'atomisation, comprenant un récipient avec une chambre à liquide pour recevoir un liquide pour atomiser, la chambre à liquide comprenant une sortie à laquelle est couplée en aval une buse de pulvérisation (50), qui est capable et configurée pour former un brouillard à partir du liquide sous une pression de fonctionnement accrue, le liquide comprenant en amont un poussoir (21) qui est reçu de manière mobile à l'intérieur et qui est capable et configuré pour expulser au cours d'un déplacement axial au moins une partie du liquide de la chambre à liquide, et le récipient pouvant au moins être couplé à des moyens d'actionnement destinés et configurés pour conférer un déplacement axial au poussoir lorsqu'il est actionné, **caractérisé par** le fait, entre le poussoir de la chambre à liquide et la buse de pulvérisation, se trouve une barrière à bulles d'air (80) qui, d'un côté orienté vers le poussoir, est au moins substantiellement infranchissable pour d'éventuelles bulles d'air dans le liquide, mais qui comprend un passage dans le but de permettre au moins substantiellement le libre passage du liquide.

2. Dispositif d'atomisation selon la revendication 1, **caractérisé par le fait que** le passage comprend au moins un canal de liquide (85) s'étendant à travers la barrière de bulles d'air d'amont en aval, au moins une paroi du canal étant hydrophile.

3. Dispositif d'atomisation selon la revendication 1 ou 2, **caractérisé par le fait qu'**au moins une paroi intérieure du récipient et une paroi extérieure de la barrière à bulles d'air sont hydrophobes à proximité d'une entrée du passage de la barrière à bulles d'air.

4. Dispositif d'atomisation selon l'une ou plusieurs des revendications précédentes, **caractérisé par le fait que** le passage comprend un canal liquide qui traverse la barrière d'amont en aval et qui débouche en amont par une entrée ayant une section transversale inférieure à 2000 microns et supérieure à 50 microns.

5. Dispositif d'atomisation selon la revendication 4, **caractérisé par le fait que** le canal de liquide s'élargit, en particulier progressivement, en direction de l'aval et s'ouvre en aval avec une section transversale supérieure à la section transversale de l'entrée sur une extrémité extérieure opposée du canal de liquide.

6. Dispositif d'atomisation selon l'une ou plusieurs des revendications précédentes, **caractérisé par le fait que** la barrière à bulles d'air est formée par un dispositif de barrière séparé, le passage comprenant un canal de liquide continu qui résiste à l'entrée de bulles d'air mais s'ouvre en amont et en aval afin de former un passage pour le liquide.

7. Dispositif d'atomisation selon la revendication 6, **caractérisé par le fait que** le dispositif de barrière est formé au moins en grande partie par un corps de barrière monolithique dans lequel s'étend le canal de liquide.

8. Dispositif d'atomisation selon la revendication 7, **caractérisé par le fait que** le corps de barrière comprend un corps au moins substantiellement solide, en particulier un corps en plastique, plus particulièrement un corps en plastique qui est hydrophile au moins dans le canal de liquide.

9. Dispositif d'atomisation selon l'une ou plusieurs des revendications 6, 7 ou 8, **caractérisé par le fait que** le dispositif de barrière comprend un bec dans lequel s'étend le canal de liquide, une pièce latérale orientée vers le bas s'étendant à partir du bec et ne s'étendant que sur une partie limitée de la longueur du bec, et permettant ici de maintenir une distance par rapport au bec.

10. Dispositif d'atomisation selon la revendication 9, **caractérisé par le fait qu'**un écran est prévu en amont près d'une entrée du canal de liquide, cet écran protégeant l'entrée du canal de liquide des bulles d'air mais laissant un espace pour le passage latéral du liquide.

11. Dispositif d'atomisation selon la revendication 6, **caractérisé par le fait que** le dispositif de barrière comprend un corps en éponge avec au moins un canal de liquide à l'intérieur, au moins un canal de liquide s'ouvrant à la fois en amont et en aval.

12. Dispositif d'atomisation selon la revendication 6, **caractérisé par le fait que** le dispositif de barrière comprend une grille avec un système d'ouvertures qui s'ouvrent en amont et en aval et qui ont chacune une section transversale inférieure à 100 microns.

13. Dispositif d'atomisation selon l'une ou plusieurs des revendications précédentes, **caractérisé par le fait que** le récipient comprend une chambre à liquide cylindrique et est particulièrement interchangeable avec un cylindre standard d'une seringue médicale.

14. Dispositif d'atomisation selon l'une ou plusieurs des revendications précédentes, **caractérisé par le fait que** le poussoir comprend une membrane étanche aux liquides mais perméable à l'air.

15. Dispositif d'atomisation selon l'une ou plusieurs des revendications précédentes, **caractérisé par le fait que** le poussoir comporte un canal d'air fermé de manière étanche au liquide.

16. Récipient avec une chambre à liquide éventuellement remplie et pourvue d'une barrière à bulles d'air pour l'application dans le dispositif d'atomisation selon l'une ou plusieurs des revendications précédentes.

17. Procédé de préparation d'un dispositif d'atomisation selon l'une ou plusieurs des revendications précédentes, un récipient destiné à recevoir un liquide à atomiser étant mis en communication ouverte avec une buse de pulvérisation en aval, une chambre à liquide dans le récipient étant remplie au moins partiellement avec le liquide et la chambre à liquide étant fermée par un poussoir mobile à l'intérieur, **caractérisé par le fait que**, avant de placer le poussoir, un dispositif de barrière étant placé en amont de la buse de pulvérisation, le dispositif de barrière étant au moins substantiellement infranchissable d'un côté de ce dispositif orienté vers le poussoir à d'éventuelles bulles d'air dans le liquide mais qui comprend un passage pour le passage au moins substantiellement libre du liquide, et qu'après que le poussoir a été placé, d'éventuelles bulles d'air dans le liquide sont entraînées vers le côté du dispositif de barrière orienté vers le poussoir.

18. Procédé selon la revendication 17, **caractérisé par le fait que** le dispositif est soumis à une centrifugeuse après que le poussoir a été disposé afin de chasser les éventuelles bulles d'air dans le liquide vers le côté du dispositif de barrière orienté vers le poussoir.

19. Procédé selon la revendication 17 ou 18, **caractérisé par le fait que** le dispositif de barrière est disposé avant que la chambre à liquide ne soit remplie de liquide.
